# EUROPÄISCHE PATENTANMELDUNG

(11) **EP 2 529 764 A1**
(43) Veröffentlichungstag der Anmeldung: **05.12.2012**
(21) Anmeldenummer: 11004466.6
(22) Anmeldetag: 31.05.2011
(51) Int. Cl.: A61L 27/42, A61L 27/56

(54) **Biologisch degradierbares kompositmaterial**

(71) Anmelder: Curasan AG, 63801 Kleinostheim (DE)
(72) Erfinder: Peters, Fabian, 68259 Mannheim (DE); Hübner, Wolf-Dietrich, 65933 Frankfurt am Main (DE); Hoffmann, Christiane, 65933 Frankfurt am Main (DE); Andic, Nikica, 65933 Frankfurt am Main (DE); Hasanovic, Kathleen, 65933 Frankfurt am Main (DE); Hniopek, Tilo, 65933 Frankfurt am Main (DE)
(74) Vertreter: Forstmeyer, Dietmar

(57) **Zusammenfassung**

Die Erfindung betrifft ein biologisch degradierbares Kompositmaterial und ein Verfahren zur Herstellung desselben. Das erfindungsgemäße biologisch degradierbare Kompositmaterial ist vorzugsweise ein Knochenaufbaumaterial, das im Bereich der regenerativen Medizin, insbesondere als temporärer Knochendefektfüller zur Knochenregeneration, eingesetzt werden kann.

## Beschreibung

### Gebiet der Erfindung

Die Erfindung betrifft ein biologisch degradierbares Kompositmaterial und ein Verfahren zur Herstellung desselben. Das erfindungsgemäße biologisch degradierbare Kompositmaterial ist vorzugsweise ein Knochenaufbaumaterial, das im Bereich der regenerativen Medizin, insbesondere als temporärer Knochendefektfüller zur Knochenregeneration, eingesetzt werden kann.

### Stand der Technik

Knochendefekte und deren Behandlung sind seit langem bekannt. Beispielsweise können Knochendefekte bei traumatischen Ereignissen (Frakturen) auftreten. Daneben können Knochendefekte auch durch Krankheiten hervorgerufen werden, beispielsweise Tumore, Knochenschwund durch Osteoporose oder durch fehlende Last wie bei der Kieferkammatrophie. In all diesen Fällen sind knöcherne Defekte vorhanden, die durch regenerative Maßnahmen behandelt werden müssen.

Das Reservoir von körpereigenem (autogenem), für eine Transplantation geeignetem Knochen ist sehr begrenzt: eine Knochenentfernung zum Aufbau an anderer Stelle kann im Beckenkammbereich, im Kinn oder Kiefernwinkel am Schienbeinkopf oder durch die Entnahme an einer Rippe oder an der Fibula erfolgen. Neben der begrenzten Verfügbarkeit, ist die Entnahme von autogenem Knochen mit den erheblichen Risiken, wie einer weiteren Operation zur Knochenentnahme sowie möglichen Folgebeschwerden an der Entnahmestelle, für den Patienten verbunden.

Neben der autogenen Knochenentnahme sind Spenderknochen (Allogene Knochen, Allografts) von humanen Spendern zu nennen. Diese Knochen werden durch aufwendige chemische Verfahren prozessiert, um sämtliches allergenes Fremdmaterial aus dem Knochen zu entfernen. Neben den Risiken von Abstoßungsreaktionen ist auch der Unterschied im Knochenmineral je nach Alter und Lebensweise der Spender zu nennen, die ein reproduzierbares Heilungsergebnis auch nach einem absolut reproduzierbaren Herstellprozess nicht garantiert. In der Publikation GRYNPAS, "Age and disease-related changes in the mineral phase of bone", Calcif. Tissue Int. 1993, 53 (1): 57-64 ist dieses beschrieben.

Als weitere Möglichkeit zum Ersatz von autogenem Knochen wurden diverse Versuche unternommen, Knochen von Säugetieren derart zu prozessieren, dass sämtliches organisches Material entfernt wird. Auch in diesen Fällen verbleibt ein Restrisiko der Immunantwort gleichermaßen wie die Möglichkeit, dass Krankheiten die Artengrenze überwinden. Durch ausgefeilte Techniken sind diese Risiken bei den so genannten xenogenen Knochenersatzmaterialien heutzutage stark minimiert.

Neben den genannten Restrisiken weisen viele Materialien biologischen Ursprungs auch ein Problem bzgl. der Röntgenopazität auf: der Behandler kann im Röntgenbild die Knochentransplantate nur schwer von der gesunden knöchernen Umgebung unterscheiden und somit die erfolgreiche und vollständige Defektfüllung sowie den Heilungsverlauf nicht immer gut kontrollieren. In EP 0 932 373 wird daher ein Knochenimplantat, bestehend aus demineralisierten und teildemineralisierten Knochenpartikeln vorgeschlagen, welches mit einem radioopaken Marker ausgestattet ist.

Zur Entwicklung alternativer Biomaterialien sind Kenntnisse im Metabolismus sowie der natürlichen Knochenauf- und -abbauprozesse notwendig. Knochen wird ständig von Osteoblasten gebildet und von Osteoklasten resorbiert. Der Abbau eines Biomaterials erfolgt sowohl durch zelluläre Resorption als auch durch hydrolytische Degradation.

In den 1960er Jahren wurden Signalproteine identifiziert (Urist, Marshall R. (1965). "Bone: formation by autoinduction". Science 12:150 (698): 893-899), die von Säugetierzellen ausgeschüttet werden, um benachbarte Zellen zu beeinflussen. Sie wirken osteoinduktiv, können also das Knochenwachstum induzieren. Diese sogenannten Bone Morphogenetic Proteins (BMPs) sowie diverse Wachstumsfaktoren wie Transforming Growth Factor Beta (TGF-β), Insulin Like Growth Factor (IGF) und andere Zytokine führen zu einem stark beschleunigten Knochenwachstum. Bemühungen gehen dahin, diese rekombinant biotechnologisch herzustellen, um sie nicht aus Knochen oder anderen Körperteilen von Säugetieren zu extrahieren. Um eine retardierende Freisetzung der Materialien zu erreichen, werden Matrices zur Verfügung gestellt, auf oder in denen die Zytokine immobilisiert sind. Derartige Matrices können beispielsweise Knochenaufbaumaterialien, Biokeramiken, organische Polymere oder auch Kollagen oder Gelatine sein.

Aufgrund der vorstehend genannten, vielfältigen Probleme, der mit dem Einsatz von autogenem Knochen, Spenderknochen oder xenogenem Knochen verbunden ist, wurde schon früh begonnen, Alternativen in Form von synthetischen Biomaterialien zu erforschen, die die Verwendung der natürlichen Knochenmaterialien ersetzen oder ergänzen können.

Bei den synthetischen Biomaterialien unterscheidet man zwischen Knochenersatz- oder Knochenaufbaumaterialien. Knochenersatzmaterial ist nicht oder nur schwer resorbierbar und bildet nach erfolgter Heilung im Defekt ein Knochen-Knochenersatzmaterial-Konstrukt. Im Gegensatz dazu sind Knochenaufbaumaterialien vollständig resorbierbar und werden im Laufe der Zeit von körpereigenem ortsständigem Knochen ersetzt. Synthetische Knochenersatz- oder Knochenaufbaumaterialien bestehen beispielsweise aus gesinterten oder aus Lösung gefällten Calciumphosphaten, beispielsweise Hydroxylapatit (HA, Ca₁₀(PO₄)₆(OH)₂) oder beta-Tricalciumphosphat (β-TCP, Ca₃(PO₄)₂); Biokeramiken, wie Biogläser oder Glaskeramiken; oder biologisch degradierbaren Kunststoffen. Typische Knochenersatzmaterialien bestehen beispielsweise aus HA oder Titan. Typische Knochenaufbaumaterialien bestehen aus β-TCP, Biogläsern, resorbierbaren Glaskeramiken oder biologisch degradierbaren Kunststoffen wie Polyestern.

Das Regenerationspotential eines Knochenaufbaumittels wird zu wesentlichen Anteilen durch seine spezielle Morphologie der Porosität bestimmt. Poren und Hohlräume sind wichtig für die Durchdringung mit Blut, Körperflüssigkeiten, für die Ernährung der Zellen und für die Auflösung/Degradation des Materials. Während ein interkonnektierendes Mikroporennetzwerk vor allem die Biokompatibilität des Materials sichert, begünstigen interkonnektierende Makroporen in einem Größenbereich von 100 bis 500 µm vor allem das knöcherne Durchbauen des Materials. In Kompositmaterialien wie auch in Granulatschüttungen muss man die intergranulären Zwischenräume ebenfalls als Pore verstanden wissen, wenn man die Abhängigkeit zum Knocheneinwachsverhalten beschreibt.

Auch die Partikelgröße und die Partikelmorphologie sind für das Regenerationspotential eines Knochenaufbaumittels essentiell. Kleine Partikel führen zu inflammatorischen, aseptischen Entzündungsreaktionen. Beispielsweise ist schon seit langer Zeit bekannt, dass Abrieb, insbesondere von den Gelenkflächen künstlicher Gelenke, zu inflammatorischen Reaktionen, hervorgerufen durch den phagocytären Abtransport der Partikel durch Fremdkörperriesenfresszellen (Makrophagen), führt. Dieser als Phagocytose bezeichnete Vorgang lässt sich vereinfacht als ein Inkorporieren der Partikel durch oder in eine Makrophage und den darauf folgenden Abtransport beschreiben. Die Makrophagocytose ist Gegenstand zahlreicher Untersuchungen. GONZALEZ et al. (O. Gonzalez, R. L. Smith, S. B. Goodman, "Effect of size, concentration, surface area and volume of polymtehylmethacrylate particles on human macrophages in vitro", Journal of Biomedical Materials Research 1996 (30), 463-473) berichten von phagocytierbaren (0,325 µm und 5,5 µm) und nichtphagocytierbaren (200 µm) Partikeln. PETERS et al. schreiben über Biomaterialien aus Beta-Tricalciumphosphat, dass die durchschnittliche Partikelgröße im Bereich von größer oder gleich 7-10 µm liegen sollte, um die Phänomene der Phagocytose zu vermeiden (F. Peters, D. Reif, "Functional Materials for Bone Regeneration from Beta-Tricalcium Phosphate", Mat.-wiss. u. Werkstofftech. 2004, 35 (4), 203-207).

Weitere wesentliche Eigenschaften von Knochenaufbaumitteln sind beispielsweise Biokompatibilität, Stabilität, insbesondere mechanische Festigkeit, Resorbierbarkeit, Gesamtporosität, Anpassungsfähigkeit an den Knochendefekt, und Handhabbarkeit für den behandelnden Arzt.

Biokompatibilität ist eine Grundvoraussetzung für Knochenaufbaumittel, denn sie bestimmt die Verträglichkeit des synthetischen Materials für das menschliche Gewebe. Für die Biokompatibilität spielen viele Faktoren eine Rolle. So ist die Oberflächenbeschaffenheit eines Knochenaufbaumittels ebenso wichtig für die Biokompatibilität, wie dessen Gesamtkonzept (z. B. Partikelgröße, Partikelmorphologie, Gesamtporosität, und Morphologie der inter- und intragranulären Porosität, d.h. Porengrößenverteilung und Interkonnektion der Poren innerhalb und zwischen den einzelnen Granulatpartikeln eines Knochenaufbaumittels) und chemische Zusammensetzung. Damit die körpereigenen Abwehrzellen das Material nicht mehr als Fremdkörper identifizieren, ist es für ein Knochenaufbaumittel unerlässlich, dass die physikalischen und chemischen Charakteristika zum Knochengewebe passen, welches ersetzt werden soll. Ein Knochenaufbaumittel wird sich allerdings immer wie ein Fremdkörper verhalten, stellt es doch keinen körpereigenen Bestandteil dar.

Zur Erhöhung der Biokompatibilität wurde beispielsweise der Zusatz von Kollagen zu den synthetischen Biomaterialien vorgeschlagen, da so ein Knochenanalog gewonnen werden kann, wenn man voraussetzt, dass Knochen aus ca. 30% Kollagen vom Typ 1 und 70% calcium-defizientem Carbonatapatit besteht. Kollagen ist biokompatibel und bildet eine bevorzugte Matrix für die Adhäsion knochenbildender Zellen (Osteoblasten). Kollagen vom Typ 1 ist die am häufigsten vorkommende Modifikation und wird am meisten im Bereich der regenerativen Medizin und bei der Gewebezüchtung verwendet. Kollagen lässt sich aus menschlichem oder tierischem Gewebe, beispielsweise Haut oder Sehnen, extrahieren. Organisch-anorganische Komposite aus Kollagen und Calciumphosphaten lässt sich durch in-vitro- Mineralisation oder durch konventionelle Misch- und Gefriertrocknungsprozesse gewinnen.

Beispielsweise wurde Kollagen im Komposit mit Hydroxylapatit beschrieben (Rodrigues et al., Characterization of a bovine collagen-hydroxyapatite composite scaffold for bone tissue engineering, Biomaterials 24, 2003, 4987-4997). Die Oberfläche des Komposits weist Arg-Gly-Asp (RGD) Proteinsequenzen auf, wodurch die Interaktion der Osteoblastenspezifischen Integrine mit der Substratoberfläche positiv beeinflusst wird. Kompositmaterialien aus Kollagen und Hydroxylapatit sowie verschiedene Herstellungsverfahren sind auch in der Publikation Wahl und Czernuska (Collagen-Hydroxyapatite Composites for Hard Tissue Repair, European Cells and Materials, 11, 2006, 43-56) beschrieben.

Zur Erhöhung der mechanischen Festigkeit wird in EP 1413321 der Einsatz einer Glaskeramik, insbesondere von Calciumalkaliorthophosphat-Glaskeramiken, z.B. mit der chemischen Zusammensetzung Ca₂KNa(PO₄)₂, vorgeschlagen. Das Material kann auch mit Silicat dotiert sein und weist neben einer erhöhten mechanischen Stabilität eine erhöhte Bioaktivität sowie lange Resorptionszeiten auf.

Zahlreiche weitere synthetische Knochenersatz- oder Knochenaufbaumaterialien, die sich in ihrem Gesamtkonzept und/oder ihrer chemischen Zusammensetzung unterscheiden, sind bekannt. Beispielsweise werden Knochenersatz- oder Knochenaufbaumaterialien beschrieben in EP 0267624, DE 29922585, DE 3717818, WO 2004/112855, EP 1413323, EP 0941130, WO 2009/00445, US 2003/009235, EP 0164483, US 4,795,467, EP 0243178, EP 0984745, DE 3414924, DE 4222763, DE 3784646, US 5,071,436, EP 0309241, EP 0386253, EP 1891984, WO 98/22154, EP 1150726, EP 1270025, US 2005/0159820, US 7,189,263, WO 02/22045, DE 102006026000, WO 03/022319, US 2008/0187571, WO 2006/031196, US 7,544,212, WO 2005/051447, WO 2006/095154, US 2006/0172918, WO 03/071991, DE 102005034421, US 2002/0133238, und EP 0302847.

Auch der Einsatz von biologisch aktiven Verbindungen in synthetischen Biomaterialien wurde bereits vorgeschlagen. So sind Knochenersatz- oder Knochenaufbaumaterialien in Verbindung mit osteogenen Proteinen, Wachstumsfaktoren, Antibiotika, und weiteren biologisch aktiven Verbindungen beispielsweise beschrieben in WO 89/09787, WO 89/09788, DE 69403439, WO 02/051449, WO 96/39202, EP 1372748, und EP 1719531.

Die Behandlung knöcherner Defekte im orthopädisch-chirurgischen Bereich und gleichermaßen von Defekten im oralen oder maxillofacialen Bereich stellt trotz der zahlreich bekannten und kommerziell verfügbaren Knochenersatz- und Knochenaufbaumaterialien nach wie vor eine große Herausforderung für den Behandler dar. Der Grund hierfür ist, dass bisher kein Material verfügbar ist, das sich den physikalischen und chemischen Charakteristika des Knochengewebes optimal anpasst und gleichzeitig über die gewünschte mechanische Festigkeit, Resorbierbarkeit, Anpassungsfähigkeit an den Knochendefekt und Handhabbarkeit für den behandelnden Arzt verfügt.

Ein Nachteil der bisher bekannten Knochenersatz- und Knochenaufbaumaterialien ist, dass sie häufig nur in einer der gewünschten Eigenschaften optimiert sind. Beispielsweise ist die Resorbierbarkeit, Anpassungsfähigkeit an den Knochendefekt und Handhabbarkeit für den behandelnden Arzt bei Knochenersatz- und Knochenaufbaumaterialien mit guter mechanischer Festigkeit zumeist eingeschränkt.

Ein weiteres Problem bekannter Knochenersatz- und Knochenaufbaumaterialien ist ihre geringe Röntgenopazität, wodurch sich der Behandlungserfolg nicht verfolgen lässt.

Auch die Morphologie der Poren und die Partikelgröße der bisher bekannten Knochenersatz- und Knochenaufbaumaterialien kann Probleme hervorrufen. So können Knochenersatz- und Knochenaufbaumaterialien, die zu lange und gewundene Poren aufweisen, nicht vollständig mit Knochen durchbaut werden. Zu lange, interkonnektierende, makroporöse Systeme bergen darüber hinaus das Risiko, dass sich Keime in die geschlossenen Enden der makroporösen Systeme einnisten können und sich dort einer systemischen Behandlung mit Antibiotika entziehen, wie beispielsweise von PALM beschrieben (Palm, F. "Cerasorb M - a new synthetic pure-phase β-TCP ceramic in oral and maxillofacial surgery", *Implants* 2006, 3 (Sept). Ferner können Knochenersatz- und Knochenaufbaumaterialien, die zu kleine Partikel aufweisen oder während der Resorption oder durch Abrieb zu zu kleinen Partikeln zerfallen, Entzündungsreaktionen hervorrufen. Da sich Knochenersatz- und Knochenaufbaumaterialien als nicht-körpereigenes Gewebe immer wie ein Fremdkörper verhalten, ist das Auftreten inflammatorischer, aseptischer Entzündungsreaktionen nach wie vor ein häufiges Problem, welches sich beim Einsatz von Knochenersatz- und Knochenaufbaumaterialien beobachten lässt.

Ziel der vorliegenden Erfindung war die Bereitstellung eines neuen biologisch degradierbaren Kompositmaterials, insbesondere eines Knochenaufbaumaterials, mit gegenüber dem Stand der Technik verbesserten Eigenschaften, wie beispielsweise vermindertem Auftreten von Fremdkörperreaktionen durch Verhindern von Phagozytose, höherer Röntgenopazität, besserer Handhabbarkeit und Anpassungsfähigkeit an den Knochendefekt.

### Beschreibung der Erfindung

Die Erfindung betrifft ein biologisch degradierbares Kompositmaterial, insbesondere ein Knochenaufbaumaterial, mit verbesserten Eigenschaften.

Hierzu stellt die vorliegende Erfindung ein biologisch degradierbares Kompositmaterial bereit, dadurch gekennzeichnet, dass das Kompositmaterial mindestens eine anorganische Komponente (a); eine anorganische Komponente (b); und mindestens eine organische Komponente (c) enthält; wobei die Dichte der anorganischen Komponente (a) und der anorganischen Komponente (b) unterschiedlich ist.

Der Begriff "biologisch degradierbar" bedeutet, dass das Kompositmaterial durch zelluläre Resorption und/oder hydrolytische Degradation im Körper eines Patient abgebaut werden kann.

Mit dem Begriff "anorganische Komponente", wie er hierin verwendet wird, ist ein biokompatibler anorganischer Werkstoff gemeint, der sich als Knochenersatz- bzw. Knochenaufbaumaterial eignet. Derartige Werkstoffe sind im Stand der Technik bekannt und umfassen beispielsweise Biokeramiken, insbesondere Calciumphosphat-Keramiken, Biogläser, enthaltend SiO₂, Na₂O, K₂O, CaO und/oder P₂O₅, Glaskeramiken, oder Mischungen derselben.

Mit dem Begriff "organische Komponente", wie er hierin verwendet wird, ist eine biokompatible organische Verbindung oder ein biokompatibler organischer Werkstoff gemeint, die in Knochenersatz- bzw. Knochenaufbaumaterial eingesetzt werden können. Derartige Verbindungen bzw. Werkstoffe sind im Stand der Technik bekannt und umfassen beispielsweise biologisch degradierbare Kunststoffe wie Polyester, Polymere aus Milchsäure oder Glycolsäure, Gelatine, Kollagen, Glycosaminoglycan, Hyaluronan, Natrium-Hyaluronat, Calciumhyaluronat, Methylcellulose, Ethylcellulose, Propylcellulose, Hydroxypropylcellulose, Hydroxyethylcellulose, Stärke, Dextran, Hydroxyethylstärke, Alginat, Polyethylenglycol, Albumin, Chitosan, Polypeptide, Proteine, und Antibiotika.

Vorzugsweise ist das biologisch degradierbare Kompositmaterial dadurch gekennzeichnet, dass die anorganische Komponente (a) in einer Menge von 2-20 Gew.-%, bezogen auf das Kompositmaterial; die anorganische Komponente (b) in einer Menge von 60-78 Gew.-%, bezogen auf das Kompositmaterial, und die organische Komponente (c) in einer Menge von 10-20 Gew.%, bezogen auf das Kompositmaterial, vorhanden ist, mit der Maßgabe, dass die Gesamtmenge der im Kompositmaterial enthaltenen Komponenten nicht mehr als 100 Gew.-% beträgt.

Das biologisch degradierbare Kompositmaterial kann ferner dadurch gekennzeichnet sein, dass die Dichte der anorganischen Komponente (a) etwa 0,8-1,5 g/cm³ und die Dichte der anorganischen Komponente (b) etwa 0,5-0,9 g/cm³ beträgt, mit der Maßgabe, dass die Dichte der anorganischen Komponente (b) kleiner ist als diejenige der anorganischen Komponente (a) und die Dichtedifferenz mindestens 0,1 g/cm³ beträgt.

Die Dichtedifferenz bezeichnet die Differenz zwischen der Dichte der anorganischen Komponente (a) und der anorganischen Komponente (b) (Dichtedifferenz = [Dichte der anorganischen Komponente (a)] - [Dichte der anorganischen Komponente (b)]).

Das biologisch degradierbare Kompositmaterial kann zusätzlich eine anorganische Komponente (d) enthalten.

Bevorzugt kann das biologisch degradierbare Kompositmaterial dadurch gekennzeichnet sein, dass die anorganische Komponente (a) und/oder die anorganische Komponente (b) und/oder, sofern vorhanden, die anorganische Komponente (d) Mikroporen und/oder Mesoporen und/oder Makroporen aufweist/aufweisen. Vorzugsweise können die Mikro-, Meso- und/oder Makroporen interkonnektieren. Stärker bevorzugt bilden die Mikroporen ein interkonnektierendes Netzwerk, in das diskrete Meso- und Makroporen homogen verteilt eingebracht sind.

Das biologisch degradierbare Kompositmaterial kann ferner bevorzugt dadurch gekennzeichnet sein, dass die Porosität der anorganischen Komponente (a), der anorganischen Komponente (b), und/oder, sofern vorhanden, der anorganischen Komponente (d) 20-85 Volumen-%, jeweils bezogen auf das Gesamtvolumen der anorganischen Komponente, beträgt.

Vorzugsweise kann das biologisch degradierbare Kompositmaterial weiterhin dadurch gekennzeichnet sein, dass die anorganische Komponente (a), die anorganische Komponente (b), und, sofern vorhanden, die anorganische Komponente (d) ein Granulat ist, welches biologisch aktive, polygon-gebrochene, abgerundete Partikel enthält, die aus Calciumphosphat und/oder Natrium- und/oder Kalium- und/oder Calcium- und/oder Silicathaltigem saurem und/oder neutralem und/oder alkalischem Bioglas, Glaskeramik oder aus Mischungen derselben bestehen.

Vorzugsweise kann das biologisch degradierbare Kompositmaterial auch dadurch gekennzeichnet sein, dass die anorganische Komponente (a), und/oder die anorganische Komponente (b), und/oder, sofern vorhanden, die anorganische Komponente (d) ein Granulat ist, welches biologisch aktive, polygon-gebrochene, abgerundete Partikel enthält, die aus Calciumphosphat mit 0 bis 25 Gew.-% Silicatglaszusatz, bezogen auf das Calciumphosphat, ohne dass eine Änderung der kristallinen Struktur des Calciumphosphat-Kristallgitters erfolgt, bestehen, wobei das Calciumphosphat ausgewählt ist aus beta-Tricalciumphosphat, vorzugsweise phasenreinem ß-Tricalciumphosphat mit 0 bis 15 Gew.-% Natrium-Magnesium-Silicatglaszusatz, Monocalciumphosphat-Monohydrat, wasserfreiem Monocalciumphosphat, Dicalciumphosphat-dihydrat, wasserfreiem Dicalciumphosphat, β-Tricalciumphosphat, Tetracalciumphosphat, Whitlockit, Octacalciumphosphat, Hydroxylapatit, Oxyapatit, Carbonatapatit vom Typ A, Carbonatapatit vom Typ B, Calcium-defizientem Hydroxylapatit, amorphem Calciumphosphat, amorphem carbonathaltigen Calciumphosphat, Calciumalkaliorthophosphat-Glaskeramiken, oder aus Mischungen derselben.

Weiterhin kann das biologisch degradierbare Kompositmaterial bevorzugt dadurch gekennzeichnet sein, dass die anorganische Komponente (a) ein Granulat mit Partikeln einer Größe von 1000-2000 µm ist, die anorganische Komponente (b) ein Granulat mit Partikeln einer Größe von 150-500 µm ist, und, sofern vorhanden, die anorganische Komponente (d) ein Granulat mit Partikeln einer Größe von 150-500 µm und einer Gesamtporosität von etwa 50-60% Volumen-%, bezogen auf die Porosität der anorganischen Komponente (a) bzw. (b), ist.

Die Gesamtporosität bezeichnet die Summe aller vorhandenen Poren. Die Porosität ist unterteilt in die intragranuläre Porosität (d.h. die in einem einzigen Granulum einer der anorganischen Komponenten (a), (b), oder (d) enthaltenen Poren) und die intergranuläre Porosität (d.h. die Poren, die die Zwischenräume zwischen den Granulaten der jeweiligen anorganischen Komponente bilden). Die Gesamtporosität wird aus Schütt- und Stampfdichte des Granulats der jeweiligen anorganischen Komponente bestimmt. Die Schüttdichte (Gewicht/Volumen) der jeweiligen anorganischen Komponente wird ermittelt, indem ein Messzylinder bis zu einem festgelegten Volumen mit der anorganischen Komponente befüllt (Volumen) und das Gewicht der anorganischen Komponente durch Wiegen bestimmt wird. Anschließend werden mittels eines Stampfvolumimeters 500 Hübe gestampft und erneut das Volumen im Messzylinder gemessen; die ermittelte Dichte (Gewicht/Volumen) ergibt die Stampfdichte.

Bevorzugt kann das biologisch degradierbare Kompositmaterial dadurch gekennzeichnet sein, dass die anorganische Komponente (a) ein Granulat mit Partikeln einer Größe von 150-500 µm und einer Dichte von 0,9-1,5 g/cm³ ist, und die anorganische Komponente (b) ein Granulat mit Partikeln einer Größe von 1000-2000 µm und einer Dichte von 0,6-0,8 g/cm³ ist.

Granuläre anorganische Biomaterialien werden im Regelfall in verschiedenen Korngrößenbereichen angeboten. Partikel mit einer bestimmten Partikelgröße (Granulatdurchmesser) sind beispielsweise durch das Zerkleinern größerer Scherben der Biokeramik und darauf folgendes Aussieben mit einer Siebkaskade erhältlich.

Bevorzugt weist die anorganische Komponente (a) eine intragranuläre Porosität (d.h. die Summe aller in einem einzigen Granulum (Partikel) enthaltenen Mikro-, Meso-, und/oder Makroporen) von 30-40 Volumen-% auf, vorzugsweise 35 Vol.-%, bezogen auf das Volumen eines einzigen Partikels der anorganischen Komponente.

Bevorzugt weist die anorganische Komponente (b) eine intragranuläre Porosität von 60-70 Volumen-% auf, vorzugsweise 60 Vol.-%, bezogen auf das Volumen eines einzigen Partikels der anorganischen Komponente.

Vorzugsweise kann das biologisch degradierbare Kompositmaterial dadurch gekennzeichnet sein, dass die anorganische Komponente (a), (b) und, sofern vorhanden, (d) ein Granulat ist, dessen Primärpartikel einen d50-Wert von mindestens 10 µm aufweisen. Der d50-Wert wird durch Laserlichtstreuung bestimmt.

Unter "Primärpartikel" werden die Ausgangspartikel der anorganischen Komponente verstanden, die durch Sintern zu Granulaten aggregiert werden; die Granulate stellen ein Agglomerat der Primärpartikel dar, in denen die Primärpartikel fest miteinander verbunden sind.

Bevorzugt kann das biologisch degradierbare Kompositmaterial dadurch gekennzeichnet sein, dass die anorganische Komponente (a), (b) und, sofern vorhanden, (d) jeweils ein Granulat aus phasenreinem beta-Tricalciumphosphat mit 0 bis 15 Gew.-% Natrium-Magnesium-Silicatglaszusatz, bezogen auf das beta-Tricalciumphosphat, oder 0 bis 100 Gew.-% Calciumalkaliorthophosphat-Glaskeramikzusatz, insbesondere Ca₂KNa(PO₄)₂, bezogen auf das beta-Tricalciumphosphat, ist.

Ferner kann das biologisch degradierbare Kompositmaterial dadurch gekennzeichnet sein, dass die mindestens eine organische Komponente (c) ausgewählt ist aus Gelatine, Kollagen, Glycosaminoglycan, Hyaluronan, Natrium-Hyaluronat, Calciumhyaluronat, Methylcellulose, Ethylcellulose, Propylcellulose, Hydroxypropylcellulose, Hydroxyethylcellulose, Stärke, Dextran, Hydroxyethylstärke, Alginat, Polyethylenglycol, Albumin, Chitosan, organisch prozessiertem oder demineralisiertem allogenem oder xenogenem Knochen, synthetischem Polypeptid, Parathormon, osteogenem Protein, oder einer Kombination derselben.

Vorzugsweise kann das biologisch degradierbare Kompositmaterial dadurch gekennzeichnet sein, dass das Kollagen der mindestens einen organischen Komponente (c) ein natives und/oder renaturiertes Kollagen ist.

Ferner kann das biologisch degradierbare Kompositmaterial bevorzugt dadurch gekennzeichnet sein, dass das Kollagen der mindestens einen organischen Komponente (c) tierischen, vorzugsweise porcinen oder bovinen Ursprungs, ist.

Bevorzugt kann das biologisch degradierbare Kompositmaterial dadurch gekennzeichnet sein, dass das Kollagen der mindestens einen organischen Komponente (c) ein Kollagen des Typs I, II oder III ist, oder eine Kombination derselben.

Ferner kann das biologisch degradierbare Kompositmaterial dadurch gekennzeichnet sein, dass das osteogene Protein der mindestens einen organischen Komponente (c) ausgewählt ist aus OP-1, OP-2, OP-3, BMP2, BMP3, BMP4, BMP5, BMP6, BMP9, BMP10, BMP11, BMP12, BMP14, BMP15, BMP16, IGF, TGF, PDGF, GDF1, GDF3, GDF5, GDF6, GDF7, GDF8, GDF9, GDF10, GDF11, oder einer Kombination derselben.

Vorzugsweise kann das biologisch degradierbare Kompositmaterial dadurch gekennzeichnet sein, dass die mindestens eine organische Komponente (c), die anorganischen Komponenten (a), (b), und, sofern vorhanden, (d) umhüllt und/oder in den Poren der anorganischen Komponenten (a), (b), und, sofern vorhanden, (d) enthalten ist und diese verstopft.

Ferner kann das biologisch degradierbare Kompositmaterial dadurch gekennzeichnet sein, dass die mindestens eine organische Komponente (c) und die anorganischen Komponenten (a), (b), und, sofern vorhanden, (d) vernetzt sind.

Das biologisch degradierbare Kompositmaterial kann auch dadurch gekennzeichnet sein, dass es weitere Komponenten, umfassend (i) eine wässrige Lösung einer hydrogelbildenden Substanz, wobei die hydrogelbildende Substanz ausgewählt ist aus modiifizierter Cellulose, Stärke, Methylcellulose, Carboxymthylcellulose, Hydroxymethylcellulose, Dextran, Hyaluronsäure, Natriumhyaluronat, Polyethylenglycol, oder einer Kombination derselben; und/oder (ii) mindestens eine antibakterielle, wundheilungsfördernde, knochenwachstumsfördernde und/oder gerinnungshemmende Substanz, in den Poren und/oder auf der Oberfläche des Kompositmaterials; als Zusatz enthält.

Bevorzugt kann das biologisch degradierbare Kompositmaterial dadurch gekennzeichnet sein, dass die Dichte des Kompositmaterials 0,1 bis 2 g/cm³, vorzugsweise 0,2 bis 0,4 g/cm³ beträgt. Die "Dichte des Kompositmaterials" bezeichnet die Schüttdichte (Gewicht/Volumen), die ermittelt wird, indem ein Messzylinder bis zu einem festgelegten Volumen mit dem Kompositmaterial befüllt und das Gewicht des Kompositmaterials durch Wiegen ermittelt wird.

Bevorzugt kann das biologisch degradierbare Kompositmaterial dadurch gekennzeichnet sein, dass die spezifische Oberfläche des Kompositmaterials 0,1 bis 2 m²/g, vorzugsweise 0,2 bis 0,4 m²/g beträgt. Die spezifische Oberfläche wird mit dem Gasadsorptionsverfahren nach Brunauer Emmett und Teller (BET) bestimmt.

Vorzugsweise kann das biologisch degradierbare Kompositmaterial dadurch gekennzeichnet sein, dass das Verhältnis zwischen Dichte und spezifischer Oberfläche des Kompositmaterials 0,1 bis 3, vorzugsweise 0,5 bis 0,7 oder 1,5 bis 2,0 beträgt.

Das biologisch degradierbare Kompositmaterial kann bevorzugt auch dadurch gekennzeichnet sein, dass das Verhältnis zwischen Dichte und Volumenschrumpfung nach Verbrennungsanalyse des Kompositmaterials 0,1 bis 1,0, vorzugsweise 0,2 bis 0,3 oder 0,5 bis 0,7 beträgt.

Das biologisch degradierbare Kompositmaterial kann in einer geometrischen Standardform, vorzugsweise als Rechteck, Quader, Zylinder oder Würfel, oder in der Form des Knochendefekts, vorzugsweise als Hohlkugelform einer Hüftpfanne oder als konische Form einer Extraktionsalveole, vorliegen.

Bevorzugt kann das biologisch degradierbare Kompositmaterial in steriler Form vorliegen.

Erfindungsgemäß umfasst das biologisch degradierbare Kompositmaterial auch alle Kombinationen der vorstehend genannten Merkmale, wobei Kombinationen bevorzugter Merkmale besonders bevorzugt sind.

Die Erfindung betrifft auch ein Verfahren zur Herstellung eines erfindungsgemäßen Kompositmaterials, umfassend die Schritte:
(S1) Zugabe der mindestens einen organischen Komponente (c) zu einer Wasser enthaltenden Lösung unter Bildung einer Suspension;
(S2) Zugabe der anorganischen Komponenten (a), (b), und, sofern vorhanden, (d); und, optional, eines Vernetzungsmittels; zur Suspension aus Schritt (S1) und Mischen der Komponenten unter Bildung einer Lösung;
(S3) Überführung der Lösung aus Schritt (S2) in eine Form und anschließende Trocknung der Mischung durch Lyophilisation unter Bildung des Kompositmaterials.

Vorzugsweise kann das erfindungsgemäße Verfahren dadurch gekennzeichnet sein, dass in Schritt (S3) nach der Lyophilisation zusätzlich eine Wärmebehandlung des Kompositmaterials bei 80 bis120 °C für 0,25 bis 48 Stunden durchgeführt wird.

Ferner kann das erfindungsgemäße Verfahren dadurch gekennzeichnet sein, dass als Vernetzungsmittel eine organische Säure, oder eine wässrige oder alkoholische Lösung einer organischen Säure, zur Suspension aus Schritt (S1) zugegeben wird, vorzugsweise Ameisensäure, Essigsäure, Propionsäure, oder eine wässrige oder alkoholische Lösung von Methansäure (Ameisensäure), Ethansäure (Essigsäure), Propansäure (Propionsäure), Butansäure (Buttersäure), n-Buttersäure, Isobuttersäure, Pentansäure (Valeriansäure), n-Valeriansäure, Iso-Valeriansäure, 2-Methylbuttersäure, Pivalinsäure, Oxalsäure, Malonsäure, Bernsteinsäure, Glutarsäure, Adipinsäure, Pimelinsäure, Hydroxyessigsäure (Glycolsäure), Hydroxypropionsäure (Milchsäure), D-Milchsäure, L-Milchsäure und deren Racemate, beta-Hydroxypropionsäure, alpha-Hydroxyvaleriansäure, beta-Hydroxyvaleriansäure, gamma-Hydroxyvaleriansäure, Hydroxymalonsäure (Tartronsäure), D-Hydroxybernsteinsäure (Äpfelsäure), L-Hydroxybernsteinsäure und deren Racemate, Dihydroxybernsteinsäure (Weinsäure) enantiomerenrein und deren Racemate (Traubensäure), Propensäure (Acrylsäure), Fumarsäure, Maleinsäure, Citronensäure, Mesoxalsäure, Acetondicarbonsäure, Oxalessigsäure, Aconitsäure, Tricarballylsäure, Ascorbinsäure, Formalydehyd, Glutaraldehyd, Genipin, Glucose, Fructose, Maltose, Dextrose, Saccharose, oder Kombinationen derselben.

Bevorzugt kann das erfindungsgemäße Verfahren dadurch gekennzeichnet sein, dass die Lösung in Schritt (S2) durch das Einleiten von Gas oder durch mechanische Manipulation geschäumt wird und Schritt (S3) mit einer geschäumten Lösung durchgeführt wird, wobei die Formgebung des Kompositmaterials vor oder nach der Lyophilisation oder der Wärmebehandlung erfolgt.

Sofern nicht anders angegeben, beziehen sich alle hierin enthaltenen Mengen- bzw. Gewichtsprozent- (Gew.%) Angaben jeweils auf das Gesamtgewicht des Kompositmaterials.

Ein Vorteil des neuen biologisch degradierbaren Kompositmaterials ist, dass damit ein flexibles Material zum Knochenaufbau oder Knochenersatz bereitgestellt wird, welches in seinen Ausführungsformen modellierbar, knetbar oder in seine ursprüngliche Form rückstellbar ist. Damit besitzt das erfindungsgemäße Kompositmaterial eine außergewöhnliche Handhabbarkeit und lässt sich maßgeschneidert an die jeweiligen Knochendefekte anpassen. Das erfindungsgemäße Kompositmaterial ist biokompatibel und osteokonduktiv, d.h. es bildet für neu gebildeten Knochen eine Leitschiene. Ferner können alle Komponenten des erfindungsgemäßen Kompositmaterials vom Organismus resorbiert, d.h. zellulär oder hydrolytisch abgebaut werden.

Von besonderem Vorteil ist, dass der Heilungsverlauf röntgenologisch verfolgbar ist, da sich das erfindungsgemäße Kompositmaterial von der biologischen Umgebung am Implantationsort durch seine höhere Röntgenopazität unterscheidet.

Ein weiterer Vorteil des erfindungsgemäßen Kompositmaterials besteht darin, dass sich die Standzeiten, d. h. die Zeit, die ein Material in einem Organismus verbleibt bevor es zu 100% resorbiert ist, der eingesetzten Komponenten unterscheiden. Dabei ist die Standzeit der mindestens einen organischen Komponente (c) kürzer als die der anorganischen Komponenten (a), (b) und gegebenenfalls (d). Idealerweise wird die anorganische Komponente durch den langsamer wachsenden Knochen innerhalb von 3 bis 6 Monaten vollständig ersetzt, wohingegen die organische Komponente nur 3-8 Wochen Standzeit aufweist. Durch die Herstelltechnik wird beispielweise eine Vernetzung der organischen Komponente ermöglicht, die steuerbar die Standzeiten der organischen Komponente von 3 bis 8 Wochen einstellen kann. Durch den inhibierenden Effekt der organischen Komponente wird erreicht, dass keine Umkristallisation der anorganischen Komponente, z.B. Calciumphospat-Phasenübergang, stattfindet.

Ferner ist das erfindungsgemäße Kompositmaterial nicht auf eine Applikation mit einem bestimmten Applikationsmittel beschränkt; es ist universell einsetzbar, d.h. mit allen im medizinischen Bereich, insbesondere dentalmedizinischen Bereich und Bereich der plastischen Chirurgie, üblichen Applikationsmitteln, wie z.B. Spatel, Skalpell, Pinzette, Bürste, Messer, Spritze- oder Kartuschensystem. Auch lässt sich das erfindungsgemäße Kompositmaterial schneiden, ohne in kleine Partikel zu zerfallen.

Das erfindungsgemäßen Kompositmaterial weist eine offen-poröse Grundstruktur auf, die zum einen blutstillende (hämostyptische) Eigenschaften hat als auch die Erschließung des erfindungsgemäßen Kompositmaterials mit Blutgefäßen (Angiogenese) und Gewebe ermöglicht. Dabei sprossen in der frühen Phase der Knochenheilung Blutgefäße und Bindegewebe in das erfindungsgemäße Kompositmaterial ein. Die Blutgefäße ermöglichen die Versorgung der Zellen und der beginnenden Gewebekompartimente mit Sauerstoff und Nährstoffen innerhalb des erfindungsgemäßen Kompositmaterials. Sobald eine hinreichend formstabile Bindegewebsstruktur vorhanden ist, ist die organische Komponente vollständig resorbiert. Das erfindungsgemäße Kompositmaterial wird durch körpereigenen, ortsständigen Knochen ersetzt, wobei es dem Knochenwachstum Vorschub leistet und das Einwachsen von schneller wachsendem Bindegewebe durch seine Platzhalterfunktion verhindert.

Ein weiterer Vorteil des erfindungsgemäßen Kompositmaterials ist sein mechanisch sehr stabiles Gefüge. Dieses Gefüge erlaubt es, die Porosität des erfindungsgemäßen Kompositmaterials zu erhöhen und seine Bioaktivität zu steigern. Ferner ist das Gefüge des erfindungsgemäßen Kompositmaterials derart stabilisiert, dass das Material erst nach längerer Behandlung mit organischer Säure angegriffen wird oder zerfällt. Eine Einwirkung der Säure im erfindungsgemäßen Zeitraum von 0 bis 6 Stunden zeigt weder Auswirkungen auf den Phasenbestand noch auf die Primärpartikelgröße. Erst nach 6 Stunden Einwirkzeit ist eine Verkleinerung der Primärkorngröße erkennbar, die aber nach wie vor die kritische Schwelle zum Auslösen von Phagozytose nicht unterschreitet.

Ein besonderer Vorteil des erfindungsgemäßen Kompositmaterials besteht darin, dass seltener Wundheilungsstörungen auftreten. So sind die Poren des erfindungsgemäßen Kompositmaterials zum Zeitpunkt der Implantation vorzugsweise verstopft, damit initial keine Keime in das Material eindringen können, die sich einer systemischen Behandlung durch Antibiotika entziehen. Bei einer bevorzugten Füllung der Poren mit Kollagen, ist es ferner möglich, dass sich ein Antibiotikum durch die offene schwammartige Kollagenstruktur bewegen kann. Weiterhin ist nach Implantation in der Akutphase der Wundheilung stets eine erhöhte makrophagocytäre Aktivität zu beobachten. Durch diese akuten Entzündungsreaktionen bedingt, können lösliche, anorganische Biokeramiken angegriffen werden und zum Zerfall in kleine Subpartikel führen. Diese kleinen Subpartikel können ihrerseits weitere phagocytäre Reaktionen auslösen und damit das Risiko einer aseptischen Fremdkörperreaktion deutlich erhöhen. Dadurch, dass das erfindungsgemäße Kompositmaterial über ein mechanisch sehr stabiles Gefüge verfügt, werden solche Prozesse vermieden. Zusätzliche Sicherheit vor verfrühtem partikulärem Zerfall wird in der ersten Zeit nach Implantation durch die bevorzugte, vollständige Umhüllung der anorganischen Komponenten durch die organische Komponente erreicht. Die Oberflächenbeschaffenheit des erfindungsgemäßen Kompositmaterials ist gleichmäßig und damit besonders biokompatibel, insbesondere wenn die organische Komponente die anorganischen Komponenten vollständig umhüllt.

### Kurze Beschreibung der Figuren

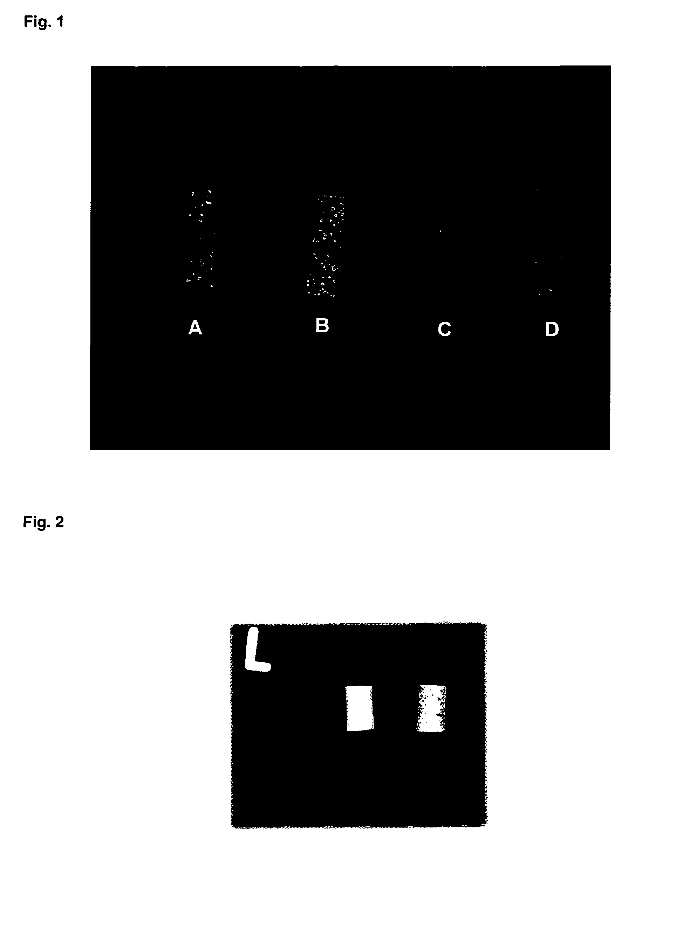

***Figur* 1****:** Vergleichende Röntgenaufnahmen von erfindungsgemäßem Kompositmaterial mit kommerziell erhältlichem Knochenersatzmaterial. A: erfindungsgemäßes Kollagen-TCP-Komposit mit der Dichte von 0,2 g/cm³, B: erfindungsgemäßes Kollagen-TCP-Komposit mit der Dichte von 0,4 g/cm³, C: VITOSS Foam Pack niedrige Dichte, D. VITOSS Foam Strip hohe Dichte. Die Beschaffenheit des erfindungsgemäßen Kompositmaterials ist gleichmäßiger und damit biokompatibler als beim kommerziellen Produkt. Weiterhin treten die Granulatkörner durch ihre Dichteunterschiede weitaus deutlicher hervor, wodurch die Röntgenopazität höher ist und das Material im Röntgenbild in der knöchernen Umgebung einfacher wiedergefunden werden kann.
***Figur* 2****:** Röntgenaufnahme in Transmission von zwei erfindungsgemäßen Gelatine-Keramik-Kompositmaterialien mit A: Korngrößen der Keramik 150-500 µm und B: 500-1000 µm. Ersichtlich ist die hohe Röntgenopazität und die gleichmäßige Oberflächenbeschaffenheit.

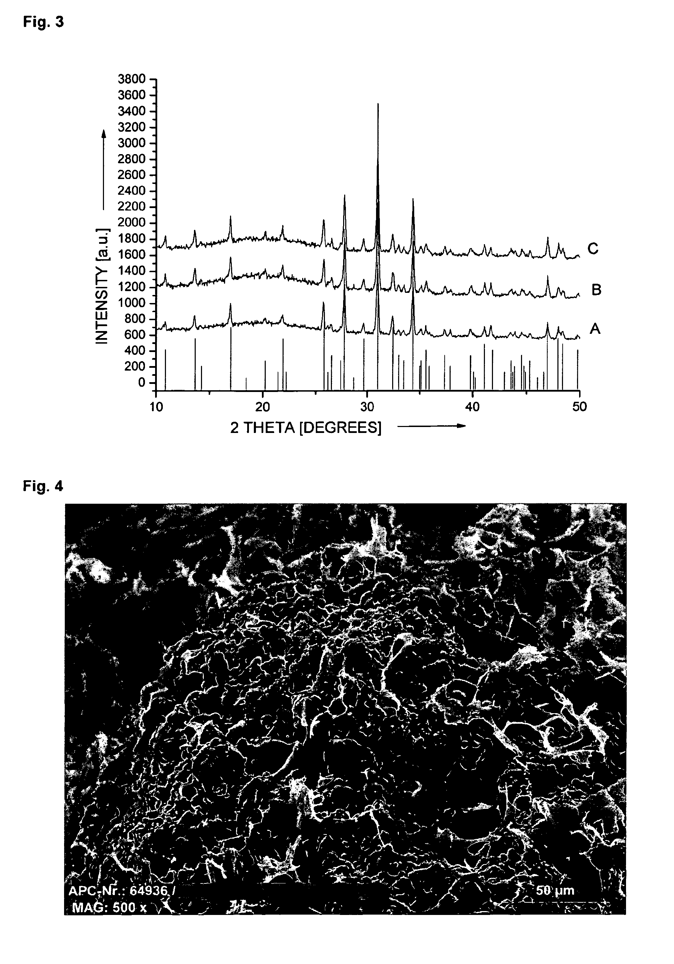

***Figur* 3****:** Röntgenpulverdiffraktogramme von erfindungsgemäßen Kollagen-TCP-Kompositen vor und nach Behandlung mit Essigsäure. Als Säulendiagramm eingezeichnet die PDF-Kartei #55-898 als Referenz für phasenreines β-Tricalciumphosphat. Der amorphe Halo ist durch die Reflexion des Kollagens verursacht. Es zeigt sich, dass die Phasenreinheit des Calciumphosphates durch die Einwirkung von Essigsäure nicht beeinflusst wird.
***Figur 4******:*** zeigt eine Rasterelektronenmikroskop-Aufnahme der keramischen Oberfläche einer Ausführungsform des erfindungsgemäßen Kompositmaterials mit Kollagen als organischer Komponente. Es ist erkennbar, dass die poröse Struktur der Keramik durch das Kollagen abgedeckt wird, das Kollagen die Poren verstopft und die Kollagenfasern die Granulate der anorganischen Komponenten vernetzen.

Nachfolgend wird die Erfindung anhand von Beispielen erläutert.

### Beispiele

Für die Herstellung der erfindungsgemäßen Kompositmaterialien werden Bestandteile A, B, C, D, E, F, G, H, I, K, L, M , N, O ,P, Q, R bereitgestellt. Dabei sind:
Bestandteil A: zu 99% phasenreines β-Tricalciumphosphat, partikulär, einer Schüttdichte von 1,1 ± 0,1 g/cm³ und einer Korngröße von <63 µm (d₅₀= 15 ± 5 µm)
Bestandteil B: bioresorbierbares Sinterglas bestehend aus 75 ± 5 Gew.-% SiO₂, 9 ± 3 Gew.-% MgO und 15 ± 5 Gew.-% Na₂O.
Bestandteil C: Schnell resorbierbare Glaskeramik der chemischen Formel Ca₂KNa(PO₄)₃ mit folgender Elementzusammensetzung: 23 ± 2 Gew.-% Ca, 11,5 ± 2 Gew.-% K, 6,5 ± 2 Gew.-% Na, 55 ± 2 Gew.-% PO₄ 2,5 ± 1 Gew.-% Mg.
Bestandteil D: Wasser für Injektionszwecke
Bestandteil E: Ammoniumhydrogencarbonat einer Korngröße von <10 µm
Bestandteil F: Ammoniumhydrogencarbonat einer Korngröße von 10-50 µm
Bestandteil G: Ammoniumhydrogencarbonat einer Korngröße von 50-500 µm
Bestandteil H: Bestandteil A, auf einem Granulierteller mit Bestandteil D unter rollenden Bewegungen stoßweise besprüht, so dass ein Aufrollen gemäß Schneeballeffekt eintritt. Nach einem 5-stündigem Sintervorgang in Höhe von 1000°C und anschließendem Sieben ist ein kugelförmiges Granulat in Fraktionen von 150-5000 µm mit einer Porosität von ungefähr 30-40% entstanden und einer Dichte von ca. 1,2 g/cm³. Die Röntgenpulverdiffraktometrie bestätigt phasenreines β-Tricalciumphosphat. Die spezifische Oberfläche, gemessen mit der BET-Methode und Krypton als Messgas ergab Werte im Bereich von 0,1-0,15 m²/g.
Bestandteil I: Bestandteil A wird mit 10 Gew.-% Bestandteil E, 20 Gew.-% Bestandteil F und 10 Gew.-% Bestandteil G innig vermischt und kalt isostatisch verpresst. Nach einem Sublimationsvorgang bei 80°C über 12 Stunden und einem anschließenden Sintervorgang bei 1000°C über 5 Stunden gefolgt von einer Zerkleinerung und Siebung. Nach anschließendem Sintervorgang bei 1000°C in 5 Stunden liegt ein Granulat mit der Größe 150-8000 µm vor, welches eine Porosität von ca. 65%, eine Schüttdichte von 0,8 g/cm³ aufweist und röntgenpulverdiffraktometrisch phasenrein ist. Die Porenverteilung ist diskret, d.h. lediglich die Mikroporen, d.h. die Poren <10 µm sind miteinander interkonnektiert. In dem interkonnektierenden mikroporösen Netzwerk sind Mesoporen (10-50 µm) und Makroporen (>50 µm) diskret verteilt und sind durch die Mikroporen für Blut und Körperflüssigkeiten zugänglich. Die Röntgenpulverdiffraktometrie bestätigt phasenreines β-Tricalciumphosphat. Messungen mit der BET-Methode und Krypton als Messgas ergab eine spezifische Oberfläche von 0,15-0,2 m²/g.
Bestandteil K: Reticulierte Polyurethan-Schwämme, hergestellt nach dem Schwartzwalder-Somers-Verfahren mit einer Porengrößenverteilung von 45 pores per inch (ppi, entspricht Porengröße von 1000 µm)
Bestandteil L: Reticulierte Polyurethan-Schwämme, hergestellt nach dem Schwartzwalder-Somers-Verfahren mit einer Porengrößenverteilung von 80 pores per inch (ppi, entspricht Porengröße von 500 µm)
Bestandteil M: Bestandteil A wird mit Bestandteil D aufgeschlämmt, 5 Gew.-% Bestandteil B hinzugegeben und feinst gemahlen, so dass ein thixotroper Schlicker mit einer Partikelgrößenverteilung von 2-5 µm (d₅₀) erreicht wird. Dieser Schlicker wird je zur Hälfte in Bestandteil K und L eingeknetet. Nach dem Sintern auf 1000°C für 5 Stunden ist der PU-Schaum restlos ausgebrannt. Zurück bleibt eine fest versinterte Keramik mit Porengrößen von 400 µm (Ausgangsbestandteil K) bzw. 250 µm (Ausgangsbestandteil L). Nach Zerkleinern, Sieben und wiederholtem Sintern unter denselben Bedingungen liegen Granulate der Korngrößen 150-8000 µm vor mit einer Gesamtporosität von 80% und einer Schüttdichte von ungefähr 0,6 g/cm³ und jeweils zur Hälfte mit den genannten Porendurchmessern. Die Röntgenpulverdiffraktometrie bestätigt phasenreines β-Tricalciumphosphat.
Bestandteil N: Bestandteil C wird mit Bestandteil D aufgeschlämmt, 5 Gew.-% Bestandteil B hinzugegeben und feinst gemahlen, so dass ein thixotroper Schlicker mit einer Partikelgrößenverteilung von 2-5 µm (d₅₀) erreicht wird. Dieser Schlicker wird je zur Hälfte in Bestandteil K und L eingeknetet. Nach dem Sintern auf 1000°C für 5 Stunden ist der PU-Schaum restlos ausgebrannt. Zurück bleibt eine fest versinterte Keramik mit Porengrößen von 400 µm (Ausgangsbestandteil K) bzw. 250 µm (Ausgangsbestandteil L). Nach Zerkleinern, Sieben und wiederholtem Sintern unter denselben Bedingungen liegen Granulate der Korngrößen 150-8000 µm vor mit einer Gesamtporosität von 80% und einer Schüttdichte von ungefähr 0,6 g/cm³ und jeweils zur Hälfte mit den genannten Porendurchmessern. Die Röntgenpulverdiffraktometrie bestätigt die Zugehörigkeit zur Phase KNaCa₂(PO₄)₂ (PDF #51-579).
Bestandteil O: Porcines Kollagen, hauptsächlich vom Typ 1, gewonnen aus dem Gewebe von kontrolliert aufgewachsenen Schweinen. Das Material ist als "für humanen Verzehr freigegeben" deklariert. Unter kontrollierten Umgebungsbedingungen werden die porcinen Gewebe prozessiert. Das Kollagen wird hoch aufgereinigt. Rückstände des Herstellprozesses sind nur noch in Spuren vorhanden und toxische Effekte damit ausgeschlossen.
Bestandteil P: Essigsäure 100%
Bestandteil Q: Porcine Gelatine gewonnen aus dem Gewebe von kontrolliert aufgewachsenen Schweinen. Das Material ist als "für humanen Verzehr freigegeben" deklariert. Unter kontrollierten Umgebungsbedingungen werden die porcinen Gewebe prozessiert.
Bestandteil R: Genipin

### Beispiel 1

Die Kornfraktion 1000-2000 µm von Bestandteil I wird ausgesiebt und jeweils 5,6 g wurden in 20 ml Bestandteil D suspendiert und 74 µl Bestandteil P zugegeben. Nach 0, 1, 3, 6, und 23 Stunden wurde das Material filtriert, gewaschen und getrocknet. Danach wurden die Partikel gewaschen, abfiltriert, getrocknet und einer Untersuchung auf Partikelgröße mit einem Laserstreuungsgerät (Fritsch Analysette), mittels Rasterelektronenmikroskopie auf die Integrität der Oberfläche sowie mittels Röntgenpulverdiffraktometrie auf Phasenreinheit untersucht. Tabelle 1 zeigt d₁₀, d₅₀ und d₉₀-Werte von Tricalciumphosphat-Pulver nach verschieden langer Einwirkzeit mit Essigsäure.

**Tabelle 1**

| Stunden in Essigsäure | d10 | d50 | d90 |
|---|---|---|---|
| 0 | 12,6 | 30,09 | 51,74 |
| 1 | 11,76 | 30,65 | 50,05 |
| 3 | 10,52 | 25,16 | 43,38 |
| 6 | 8,74 | 24,06 | 42,31 |
| 23 | 9,79 | 21,98 | 37,27 |

Wie Tabelle 1 zu entnehmen ist, erfolgt erst nach 6 Stunden eine nennenswerte Verkleinerung der Korngrößen, wobei der Feinanteil, der durch den d10-Wert repräsentiert wird, einer stärkeren Degradation unterliegt wie der partikuläre Zerfall der gröberen Anteile. Demnach ist die Flächenätzung der kleinen Partikel mit größeren Oberflächen als stärkerer Einfluss anzusehen als die Korngrenzenätzung. Auch nach 23 Stunden ist der Feinanteil nicht stark unter die kritische Schwelle des phagocytierbaren Bereiches getreten, so dass ein erhöhtes Risiko überschießender Fremdkörperreaktionen durch die Implantation des Bestandteils I in Knochen oder Gewebe auch nach längerer Säurebehandlung auszuschließen ist.

Die Ergebnisse der Röntgenpulverdiffraktometrie bezüglich der Phasenreinheit sind in ***Figur 3*** gezeigt. Wie ***Figur 3*** zu entnehmen ist, hat die Einwirkung von Essigsäure auch keinen Einfluss auf die Phasenreinheit, so dass sowohl ein erhöhtes Risiko überschießender Fremdkörperreaktionen durch die Implantation des Bestandteils I in Knochen oder Gewebe als auch eine Verminderung der Resorbierbarkeit durch Umwandlung in eine nichtresorbierbare Calciumphosphatphase nach längerer Säureeinwirkung auszuschließen ist.

### Beispiel 2

16,5 g Bestandteil O werden in 3,5 ml Bestandteil D gegeben und mittels einer Kolloidmühle suspendiert. Die Kornfraktion 1000-2000 µm von Bestandteil I wird ausgesiebt und 5,6 g wurden in die Suspension gegeben. Nach Zugabe von 74 µl Bestandteil P wird das Material bei Raumtemperatur gerührt. Nach 0, 30, 120 und 165 Minuten wird die Säure neutralisiert, das Gemisch in Form gegossen und gefriergetrocknet. Mittels Röntgenpulverdiffraktometrie wird geprüft ob die Einwirkzeit der Säure einen Einfluss auf den Phasenbestand des Calciumphosphats hatte. Die Ergebnisse zeigen, dass die Einwirkung der Säure keinen Einfluss auf den Phasenbestand des Materials hatte.

### Beispiel 3

Aus Bestandteil H wird die Granulatfraktion 150-500 µm herausgesiebt. Aus Bestandteil I werden die Fraktionen 150-500 µm sowie 1000-2000 µm herausgesiebt. Diese Komponenten werden in verschiedenen Verhältnissen gemäß ***Tab*e*lle* 2** vermischt. 190 g Bestandteil O wird in 1000 ml Bestandteil D suspendiert und mittels der Kolloidmühle vermahlen. 760 g des Granulatgemisches werden hinzugegeben und die Mischung innig gerührt. Nach Zugabe von 10 ml Bestandteil B wird die Mischung neutralisiert, in eine quadratische Edelstahlform gegossen und lyophilisiert. Je nach Vorgehen bei der Lyophilisation (langsam oder schnell) können Kompositmaterialien mit Dichten von 0,2 oder 0,4 g/ml erreicht werden. Tabelle 2 gibt über die verschiedenen Mischungen und die gemessenen Dichten und spezifischen Oberflächen Auskunft.

**Tabelle 2: Messergebnisse verschiedener Kollagen-Keramik-Komposite im Vergleich mit Kollagen**

| Probe | Bestandteil H 150-500 µm | Bestandteil I 150-500 µm | Bestandteil I 1000-2000 | Kollagen | Dichte | Spezifische Oberfläche | Verhältnis Dichte zu Oberfläche |
|---|---|---|---|---|---|---|---|
| | [Ma.-%] | [Ma.-%] | µm [Ma.-%] | [Ma.-%] | [g/ml] | [m²/g] | |
| CF-33 | 4 | 4 | 72 | 20 | 0,4 | 0,2605 | 1,54 |
| CF-34 | 2 | 6 | 72 | 20 | 0,4 | 0,25 | 1,6 |
| CF-35 | 4 | 12 | 64 | 20 | 0,4 | 0,2199 | 1,82 |
| CF-36 | 0 | 20 | 60 | 20 | 0,4 | 0,2152 | 1,86 |
| CF-37 | 4 | 4 | 72 | 20 | 0,2 | 0,3658 | 0,32 |
| CF-38 | 2 | 6 | 72 | 20 | 0,2 | 0,3092 | 0,65 |
| CF-39 | 4 | 12 | 64 | 20 | 0,2 | 0,306 | 0,66 |
| CF-40 | 0 | 20 | 60 | 20 | 0,2 | 0,3547 | 0,56 |
| Kollagen | 0 | 0 | 0 | 100 | 0,3 | 1,5768 | 0,19 |

Es zeigt sich, dass 100% reines Kollagen eine 5 bis 8-fach größere Oberfläche aufweist. Die Porosität wird also nachhaltig gesenkt. Die Poren der Keramik sind zunächst nicht zugänglich, da das Kollagen in diese eingedrungen ist und die poröse Keramikstruktur durchzogen hat (siehe beispielsweise ***Figur 4***)*.* Durch die Zugabe der partikulären Calciumphosphate lässt sich das Verhältnis zwischen Dichte und spezifischer Oberfläche über weite Strecken einstellen.

### Beispiel 4

Zur Bestimmung der Volumenschrumpfung und zur Kontrolle der Homogenität wurden die erfindungsgemäßen Kompositmaterialien gemäß Beispiel 3 einer Veraschungsanalyse mit Dichtebestimmung unterworfen. Dabei wurden aus den Kompositen Formkörper ausgeschnitten, deren Volumen und Gewicht bestimmt, und daraus die Dichte ermittelt. Anschließend wurden die Formkörper in einen Tiegel überführt und in einem Ofen die organische Phase der Formkörper restlos verbrannt. Zurück bleibt die Keramik, deren Gewicht und Schüttdichte nach dem Abkühlen bestimmt wurde. Aus dieser Analyse lassen sich die Volumenprozente der organischen und anorganischen Komponenten bestimmen und das verbleibende Volumen nach der schnelleren Resorption. Die Ergebnisse der Untersuchungen sind in Tabelle 3 zusammengefasst. Zum Vergleich mit den erfindungsgemäßen Kompositmaterialien wurden auch die zwei kommerziell erhältlichen Kollagen/TCP-Komposite "Vitoss Foam Strip" und "Vitoss Foam Pack" untersucht.

**Tabelle 3: Volumenschrumpfung der Kompositmaterialien nach Veraschungsanalyse**

| | Volumen [cm^{3]} | Gewicht nach Ausbrennen [g] | Stampfdichte nach Ausbrennen [g/cm³] | Volumen nach Ausbrennen [cm³] | Vol.-% gemessen zum Ausgangs-volumen | Schrumpfung in % |
|---|---|---|---|---|---|---|
| CF 33 | 2,18 | 0,57 | 0,79 | 0,72 | 33,0 | 67,0 |
| CF 34 | 2,58 | 0,68 | 0,76 | 0,89 | 34,6 | 65,4 |
| CF 35 | 2,37 | 0,67 | 0,76 | 0,88 | 36,9 | 63,1 |
| CF 36 | 2,51 | 0,63 | 0,73 | 0,87 | 34,6 | 65,4 |
| CF 37 | 2,51 | 0,46 | 0,69 | 0,67 | 26,6 | 73,4 |
| CF 38 | 2,41 | 0,45 | 0,66 | 0,68 | 28,1 | 71,9 |
| CF 39 | 2,00 | 0,34 | 0,67 | 0,50 | 25,0 | 75,0 |
| CF 40 | 2,26 | 0,41 | 0,84 | 0,49 | 21,8 | 78,2 |
| Vitoss Foam Strip | 1,43 | 0,36 | 0,32 | 1,14 | 79,4 | 20,6 |
| Vitoss Foam Pack | 1,74 | 0,32 | 0,39 | 0,82 | 46,9 | 53,1 |

Die Volumenschrumpfung erfindungsgemäßer Kompositmaterialien mit einer Dichte von 0,2 g/cm³ beträgt nach dem Ausbrennen der Kollagene 70-80%. Die Volumenschrumpfung erfindungsgemäßer Kompositmaterialien mit einer Dichten von 0,4 g/cm³ beträgt nach dem Ausbrennen 60-70%. Für das Verhältnis zwischen Dichte und Schrumpfung ergeben sich für die erfindungsgemäßen Kompositmaterialien damit Werte von 0,2 bis 0,6. Im Gegensatz dazu zeigen die kommerziell erhältlichen Kollagen/TCP-Komposite eine deutlich geringere Volumenschrumpfung nach dem Ausbrennen, nämlich 21% bei "Vitoss Foam Strip" und 53% bei "Vitoss Foam Pack". Das erfindungsgemäße Kompositmaterial enthält einen höheren Kollagenanteil. Nach Implantation resorbiert die organische Komponente (z.B. Kollagen) schneller als die anorganische in knöcherner Umgebung. Die größere Volumenabnahme des erfindungsgemäßen Kompositmaterials geht deshalb auch mit einer schnelleren Regeneration und damit einem schnelleren Behandlungserfolg einher.

### Beispiel 5

2,25g Bestanteil Q werden mit 15ml Bestandteil D vermengt und unter Rühren auf 70°C erwärmt. Nach dem vollständigen Lösen des Bestanteils Q, und dem Abkühlen der Lösung auf 40°C werden zuerst 0,0225g Bestanteil R und dann 13,5 g der Granulatfraktionen150-500 µm des Bestandteiles I sowie 1 g der Fraktion <150 µm derselben Substanz hinzugegeben. Die noch warme Lösung wird anschließend in eine Form gegossen und zum Auskühlen und Vernetzten in den Kühlschrank gestellt. Ein Entformen der Implantate kann nach 12 h durchgeführt werden. Nach dem Verpacken kann eine Gamma-Sterilisation erfolgen.

### Beispiel 6

1,5g Bestandteil Q werden mit 15ml Bestandteil D vermengt und unter Rühren auf 70°C erwärmt. Nach dem vollständigen Lösen des Bestandteils A, werden 0,0225g Bestanteil R, 20g der Granulatfraktion <150 µm des Bestandteiles I und 2,5g der Granulatfraktion 150-500 µm des Bestandteiles I zugegeben. In die Lösung wird folgend Luft eingeblasen und das geschäumte Material in eine Form gegossen. Zum Erkalten und Vernetzten wird die Form in den Kühlschrank gestellt. Ein Entformen der Implantate kann nach 12 h durchgeführt werden. Nach dem Verpacken kann eine Sterilisation durch Gamma-Strahlen erfolgen.

### Beispiel 7

Aus Bestandteil M wird die Granulatfraktion 150-500 µm sowie die Fraktion 1000-4000 µm herausgesiebt und im Verhältnis 1:1 gemischt. 190 g Bestandteil O wird in 1000 ml Bestandteil D suspendiert und mittels der Kolloidmühle vermahlen. Das Gemisch aus den beiden Granulatfraktionen wird hinzugegeben und die Mischung innig gerührt. Nach Zugabe von 10 ml Bestandteil B wird die Mischung neutralisiert, in eine quadratische Edelstahlform gegossen und lyophilisiert. Je nach Vorgehen bei der Lyophilisation (langsam oder schnell) können Kompositmaterialien mit Dichten von 0,2 oder 0,4 g/ml erreicht werden. Nach dem Verpacken kann eine Sterilisation durch Gamma-Strahlen erfolgen.

### Beispiel 8

Aus Bestandteil M wird die Granulatfraktion 150-500 µm sowie die Fraktion 1000-4000 µm herausgesiebt und im Verhältnis 1:1 gemischt. 190 g Bestandteil O wird in 1000 ml Bestandteil D suspendiert und mittels der Kolloidmühle vermahlen. Das Gemisch aus den beiden Granulatfraktionen wird hinzugegeben und die Mischung innig gerührt. Nach Zugabe von 10 ml Bestandteil B wird die Mischung neutralisiert, in eine quadratische Edelstahlform gegossen und lyophilisiert. Je nach Vorgehen bei der Lyophilisation (langsam oder schnell) können Kompositmaterialien mit Dichten von 0,2 oder 0,4 g/ml erreicht werden. Nach dem Verpacken kann eine Sterilisation durch Gamma-Strahlen erfolgen

## Patentansprüche

1. Biologisch degradierbares Kompositmaterial, **dadurch gekennzeichnet, dass** das Kompositmaterial mindestens eine anorganische Komponente (a); eine anorganische Komponente (b); und mindestens eine organische Komponente (c) enthält; wobei die Dichte der anorganischen Komponente (a) und der anorganischen Komponente (b) unterschiedlich ist.

2. Biologisch degradierbares Kompositmaterial nach Anspruch 1, **dadurch gekennzeichnet, dass** die anorganische Komponente (a) in einer Menge von 2-20 Gew.-%, bezogen auf das Kompositmaterial; die anorganische Komponente (b) in einer Menge von 60-78 Gew.-%, bezogen auf das Kompositmaterial, und die organische Komponente (c) in einer Menge von 10-20 Gew.%, bezogen auf das Kompositmaterial, vorhanden ist, mit der Maßgabe, dass die Menge der im Kompositmaterial enthaltenen Komponenten nicht mehr als 100 Gew.-% beträgt.

3. Biologisch degradierbares Kompositmaterial nach Anspruch 1 oder Anspruch 2, **dadurch gekennzeichnet, dass** die Dichte der anorganischen Komponente (a) etwa 0,8-1,5 g/cm³ und die Dichte der anorganischen Komponente (b) etwa 0,5-0,9 g/cm³ beträgt, mit der Maßgabe, dass die Dichte der anorganischen Komponente (b) kleiner ist als diejenige der anorganischen Komponente (a) und die Dichtedifferenz mindestens 0,1 beträgt.

4. Biologisch degradierbares Kompositmaterial nach irgendeinem der Ansprüche 1 bis 3, **dadurch gekennzeichnet, dass** das Kompositmaterial zusätzlich eine anorganische Komponente (d) enthält.

5. Biologisch degradierbares Kompositmaterial nach irgendeinem der Ansprüche 1 bis 4, **dadurch gekennzeichnet, dass** die anorganische Komponente (a) und/oder die anorganische Komponente (b) und/oder, sofern vorhanden, die anorganische Komponente (d) Mikroporen und/oder Mesoporen und/oder Makroporen aufweist/aufweisen.

6. Biologisch degradierbares Kompositmaterial nach Anspruch 5, **dadurch gekennzeichnet, dass** die Mikro-, Meso- und/oder Makroporen interkonnektieren.

7. Biologisch degradierbares Kompositmaterial nach Anspruch 5 oder Anspruch 6, **dadurch gekennzeichnet, dass** die Mikroporen ein interkonnektierendes Netzwerk bilden, in das diskrete Meso- und Makroporen homogen verteilt eingebracht sind.

8. Biologisch degradierbares Kompositmaterial nach irgendeinem der Ansprüche 1 bis 7, **dadurch gekennzeichnet, dass** die Porosität der anorganischen Komponente (a), der anorganischen Komponente (b), und/oder, sofern vorhanden, der anorganischen Komponente (d) 20-85 Volumen-%, jeweils bezogen auf das Gesamtvolumen der anorganischen Komponente, beträgt.

9. Biologisch degradierbares Kompositmaterial nach irgendeinem der Ansprüche 1 bis 8, **dadurch gekennzeichnet, dass** die anorganische Komponente (a), die anorganische Komponente (b), und, sofern vorhanden, die anorganische Komponente (d) ein Granulat ist, welches biologisch aktive, polygon-gebrochene, abgerundete Partikel enthält, die aus Calciumphosphat und/oder Natrium- und/oder Kalium- und/oder Calcium- und/oder Silicathaltigem saurem und/oder neutralem und/oder alkalischem Bioglas, Glaskeramik oder aus Mischungen derselben bestehen.

10. Biologisch degradierbares Kompositmaterial nach irgendeinem der Ansprüche 1 bis 9, **dadurch gekennzeichnet, dass** die anorganische Komponente (a), und/oder die anorganische Komponente (b), und/oder, sofern vorhanden, die anorganische Komponente (d) ein Granulat ist, welches biologisch aktive, polygon-gebrochene, abgerundete Partikel enthält, die aus Calciumphosphat mit 0 bis 25 Gew.-% Silicatglaszusatz, bezogen auf das Calciumphosphat, ohne dass eine Änderung der kristallinen Struktur des Calciumphosphat-Kristallgitters erfolgt, bestehen, wobei das Calciumphosphat ausgewählt ist aus beta-Tricalciumphosphat, vorzugsweise phasenreinem ß-Tricalciumphosphat mit 0 bis 15 Gew.-% Natrium-Magnesium-Silicatglaszusatz, Monocalciumphosphat-Monohydrat, wasserfreiem Monocalciumphosphat, Dicalciumphosphat-dihydrat, wasserfreiem Dicalciumphosphat, β-Tricalciumphosphat, Tetracalciumphosphat, Whitlockit, Octacalciumphosphat, Hydroxylapatit, Oxyapatit, Carbonatapatit vom Typ A, Carbonatapatit vom Typ B, Calcium-defizientem Hydroxylapatit, amorphem Calciumphosphat, amorphem carbonathaltigen Calciumphosphat, Calciumalkaliorthophosphat-Glaskeramik, oder aus Mischungen derselben.

11. Biologisch degradierbares Kompositmaterial nach irgendeinem der Ansprüche 1 bis 10, **dadurch gekennzeichnet, dass** die anorganische Komponente (a) ein Granulat mit Partikeln einer Größe von 1000-2000 µm ist, die anorganische Komponente (b) ein Granulat mit Partikeln einer Größe von 150-500 µm ist, und, sofern vorhanden, die anorganische Komponente (d) ein Granulat mit Partikeln einer Größe von 150-500 µm und einer Gesamtporosität von etwa 50-60% Volumen-%, bezogen auf die Porosität der anorganischen Komponente (a) bzw. (b), ist.

12. Biologisch degradierbares Kompositmaterial nach irgendeinem der Ansprüche 1 bis 10, **dadurch gekennzeichnet, dass** die anorganische Komponente (a) ein Granulat mit Partikeln einer Größe von 150-500 µm und einer Dichte von 0,9-1,5 g/cm³ ist, und die anorganische Komponente (b) ein Granulat mit Partikeln einer Größe von 1000-4000 µm und einer Dichte von0,6-0,8 g/cm³ ist.

13. Biologisch degradierbares Kompositmaterial nach irgendeinem der Ansprüche 1 bis 12, **dadurch gekennzeichnet, dass** die anorganische Komponente (a), (b) und, sofern vorhanden, (d) ein Granulat ist, dessen Primärpartikel einen d50-Wert von mindestens 10 µm aufweisen.

14. Biologisch degradierbares Kompositmaterial nach irgendeinem der Ansprüche 1 bis 13, **dadurch gekennzeichnet, dass** die anorganische Komponente (a), (b) und, sofern vorhanden, (d) jeweils ein Granulat aus phasenreinem beta-Tricalciumphosphat mit 0 bis 15 Gew.-% Natrium-Magnesium-Silicatglaszusatz, bezogen auf das beta-Tricalciumphosphat, oder 0 bis 100 Gew.-% Calciumalkaliorthophosphat-Glaskeramikzusatz, insbesondere Ca₂KNa(PO₄)₂, bezogen auf das beta-Tricalciumphosphat, ist.

15. Biologisch degradierbares Kompositmaterial nach irgendeinem der Ansprüche 1 bis 14, **dadurch gekennzeichnet, dass** die mindestens eine organische Komponente (c) ausgewählt ist aus Gelatine, Kollagen, Glycosaminoglycan, Hyaluronan, Natrium-Hyaluronat, Calciumhyaluronat, Methylcellulose, Ethylcellulose, Propylcellulose, Hydroxypropylcellulose, Hydroxyethylcellulose, Stärke, Dextran, Hydroxyethylstärke, Alginat, Polyethylenglycol, Albumin, Chitosan, organisch prozessiertem oder demineralisiertem allogenem oder xenogenem Knochen, synthetischem Polypeptid, Parathormon, osteogenem Protein, oder einer Kombination derselben.

16. Biologisch degradierbares Kompositmaterial nach Anspruch 15, **dadurch gekennzeichnet, dass** das Kollagen ein natives und/oder renaturiertes Kollagen ist.

17. Biologisch degradierbares Kompositmaterial nach Anspruch 15, **dadurch gekennzeichnet, dass** das Kollagen tierischen, vorzugsweise porcinen oder bovinen Ursprungs, ist.

18. Biologisch degradierbares Kompositmaterial nach irgendeinem der Ansprüche 15 bis 17, **dadurch gekennzeichnet, dass** das Kollagen ein Kollagen des Typs I, II oder III ist, oder eine Kombination derselben.

19. Biologisch degradierbares Kompositmaterial nach Anspruch 15, **dadurch gekennzeichnet, dass** das osteogene Protein ausgewählt ist aus OP-1, OP-2, OP-3, BMP2, BMP3, BMP4, BMP5, BMP6, BMP9, BMP10, BMP11, BMP12, BMP14, BMP15, BMP16, IGF, TGF, PDGF, GDF1, GDF3, GDF5, GDF6, GDF7, GDF8, GDF9, GDF10, GDF11, oder einer Kombination derselben.

20. Biologisch degradierbares Kompositmaterial nach irgendeinem der Ansprüche 1 bis 19, **dadurch gekennzeichnet, dass** die organische Komponente (c), die anorganischen Komponenten (a), (b), und, sofern vorhanden, (d) umhüllt und/oder in den Poren der anorganischen Komponenten (a), (b), und, sofern vorhanden, (d) enthalten ist und diese verstopft.

21. Biologisch degradierbares Kompositmaterial nach irgendeinem der Ansprüche 1 bis 20, **dadurch gekennzeichnet, dass** die organische Komponente (c) und die anorganischen Komponenten (a), (b), und, sofern vorhanden, (d) vernetzt sind.

22. Biologisch degradierbares Kompositmaterial nach irgendeinem der Ansprüche 1 bis 21, **dadurch gekennzeichnet, dass** es weitere Komponenten, umfassend (i) eine wässrige Lösung einer hydrogelbildenden Substanz, wobei die hydrogelbildende Substanz ausgewählt ist aus modiifizierter Cellulose, Stärke, Methylcellulose, Carboxymthylcellulose, Hydroxymethylcellulose, Dextran, Hyaluronsäure, Natriumhyaluronat, Polyethylenglycol, oder einer Kombination derselben; und/oder (ii) mindestens eine antibakterielle, wundheilungsfördernde, knochenwachstumsfördernde und/oder gerinnungshemmende Substanz, in den Poren und/oder auf der Oberfläche des Kompositmaterials; als Zusatz enthält.

23. Biologisch degradierbares Kompositmaterial nach irgendeinem der Ansprüche 1 bis 22, **dadurch gekennzeichnet, dass** die Dichte des Kompositmaterials 0,1 bis 2 g/cm³, vorzugsweise 0,2 bis 0,4 g/cm³ beträgt.

24. Biologisch degradierbares Kompositmaterial nach irgendeinem der Ansprüche 1 bis 23, **dadurch gekennzeichnet, dass** die spezifische Oberfläche des Kompositmaterials 0,1 bis 2 m²/g, vorzugsweise 0,2 bis 0,4 m²/g beträgt.

25. Biologisch degradierbares Kompositmaterial nach irgendeinem der Ansprüche 1 bis 24, **dadurch gekennzeichnet, dass** das Verhältnis zwischen Dichte und spezifischer Oberfläche des Kompositmaterials 0,1 bis 3, vorzugsweise 0,5 bis 0,7 oder 1,5 bis 2,0 beträgt.

26. Biologisch degradierbares Kompositmaterial nach irgendeinem der Ansprüche 1 bis 25, **dadurch gekennzeichnet, dass** das Verhältnis zwischen Dichte und Volumenschrumpfung nach Verbrennungsanalyse des Kompositmaterials 0,1 bis 1,0, vorzugsweise 0,2 bis 0,3 oder 0,5 bis 0,7 beträgt.

27. Biologisch degradierbares Kompositmaterial nach irgendeinem der Ansprüche 1 bis 26, **dadurch gekennzeichnet, dass** das Kompositmaterial in einer geometrischen Standardform, vorzugsweise als Rechteck, Quader, Zylinder oder Würfel, oder in der Form des Knochendefekts, vorzugsweise als Hohlkugelform einer Hüftpfanne oder als konische Form einer Extraktionsalveole, vorliegt.

28. Biologisch degradierbares Kompositmaterial nach irgendeinem der Ansprüche 1 bis 27, **dadurch gekennzeichnet, dass** das Kompositmaterial in steriler Form vorliegt.

29. Verfahren zur Herstellung eines Kompositmaterials gemäß irgendeinem der Ansprüche 1 bis 28, umfassend die Schritte:
(S1) Zugabe der mindestens einen organischen Komponente (c) zu einer Wasser enthaltenden Lösung unter Bildung einer Suspension;
(S2) Zugabe der anorganischen Komponenten (a), (b), und, sofern vorhanden, (d); und, optional, eines Vernetzungsmittels; zur Suspension aus Schritt (S1) und Mischen der Komponenten unter Bildung einer Lösung;
(S3) Überführung der Lösung aus Schritt (S2) in eine Form und anschließende Trocknung der Mischung durch Lyophilisation unter Bildung des Kompositmaterials.

30. Das Verfahren nach Anspruch 29, **dadurch gekennzeichnet, dass** in Schritt (S3) nach der Lyophilisation zusätzlich eine Wärmebehandlung des Kompositmaterials bei 80 bis120 °C für 0,25 bis 48 Stunden durchgeführt wird.

31. Das Verfahren nach Anspruch 29 oder Anspruch 30, **dadurch gekennzeichnet, dass** als Vernetzungsmittel eine organische Säure, oder eine wässrige oder alkoholische Lösung einer organischen Säure, zur Suspension aus Schritt (S1) zugegeben wird, vorzugsweise Ameisensäure, Essigsäure, Propionsäure, oder eine wässrige oder alkoholische Lösung von Methansäure (Ameisensäure), Ethansäure (Essigsäure), Propansäure (Propionsäure), Butansäure (Buttersäure), n-Buttersäure, Isobuttersäure, Pentansäure (Valeriansäure), n-Valeriansäure, Iso-Valeriansäure, 2-Methylbuttersäure, Pivalinsäure, Oxalsäure, Malonsäure, Bernsteinsäure, Glutarsäure, Adipinsäure, Pimelinsäure, Hydroxyessigsäure (Glycolsäure), Hydroxypropionsäure (Milchsäure), D-Milchsäure, L-Milchsäure und deren Racemate, beta-Hydroxypropionsäure, alpha-Hydroxyvaleriansäure, beta-Hydroxyvaleriansäure, gamma-Hydroxyvaleriansäure, Hydroxymalonsäure (Tartronsäure), D-Hydroxybernsteinsäure (Äpfelsäure), L-Hydroxybernsteinsäure und deren Racemate, Dihydroxybernsteinsäure (Weinsäure) enantiomerenrein und deren Racemate (Traubensäure), Propensäure (Acrylsäure), Fumarsäure, Maleinsäure, Citronensäure, Mesoxalsäure, Acetondicarbonsäure, Oxalessigsäure, Aconitsäure, Tricarballylsäure, Ascorbinsäure, Formalydehyd, Glutaraldehyd, Genipin, Glucose, Fructose, Maltose, Dextrose, Saccharose, oder Kombinationen derselben.

32. Das Verfahren nach irgendeinem der Ansprüche 29 bis 31, **dadurch gekennzeichnet, dass** die Lösung in Schritt (S2) durch das Einleiten von Gas oder durch mechanische Manipulation geschäumt wird und Schritt (S3) mit einer geschäumten Lösung durchgeführt wird, wobei die Formgebung des Kompositmaterials vor oder nach der Lyophilisation oder der Wärmebehandlung erfolgt.
